# EUROPEAN PATENT APPLICATION

(11) **EP 4 537 758 A1**
(43) Date of publication of application: **16.04.2025**
(21) Application number: 23383034.8
(22) Date of filing: 09.10.2023
(51) Int. Cl.: A61B 6/03, A61B 6/00

(54) **IMAGING SYSTEM AND METHODS, DEVICES FOR USE IN A PET SCANNER**

(71) Applicant: Institut de Fisica d'Altes Energies (IFAE), 08193 Bellaterra, Barcelona (ES)
(72) Inventor: CHMEISSANI RAAD, Mokhtar, 08019 Barcelona (ES)
(74) Representative: Bardehle Pagenberg S.L.

(57) **Abstract**

The present disclosure relates to a device for use in a PET scanner, comprising a base configured to be worn around a head of a subject, wherein the base comprises three or more radioactive point sources which are non-pure positron emitters which simultaneously emit a positron that annihilates into two back-to-back gamma photons with 511 keV and into an additional gamma photon of different energy. The present disclosure further relates to a controller for a PET scanner configured to receive data from one or more photon detectors, and to determine positions of the radioactive point sources based on the sensor events. The present disclosure further relates to a PET scanner, to methods for monitoring movements of a head of a subject in a PET scanner and to methods for scanning a brain of a subject.

## Description

The present disclosure relates to medical imaging, and more particularly the present disclosure relates to device for use in PET scanners. The present disclosure relates to devices and methods for monitoring movements of a subject, and more particularly the head of the subject, during a PET scan. The present disclosure further relates to methods for scanning metabolic activity in the brain of the subject.

### BACKGROUND

Dementia is an umbrella term for degenerative neurological diseases. Dementia is on the rise among the aging population in industrial countries in general and in the EU countries in particular. Currently, it is estimated that every 3 seconds a new case of dementia is registered worldwide. Annually, this means there are about 10 million people diagnosed with dementia, of which two third are women. It is estimated that in 2050 this number will increase to 27 million. About 50% to 60% of cases of dementia can be attributed to Alzheimer's disease (AD).

A well-known method of medical imaging used for the diagnosis of brain diseases such as dementia is Positron Emission Tomography (PET). Before a PET scan, a patient is administered a radioactive drug called a "tracer". The tracer travels through the patient's circulatory system and accumulates in several organs of the body. Within the different tracers, amyloid PET radiotracers such as 91 flutemetamol have been found to have the potential to improve PET brain imaging with the purpose of detecting Alzheimer's disease in early stages.

Radioactive isotopes decay via the β⁺ process with the emission of positrons. The positron annihilates with an electron from the patient's body and their masses are transformed into two almost co-linear gamma-photons travelling in opposite directions (two back-to-back emitted gamma-photons), each with an energy of 511 keV. The PET scanner registers the position of the emitted photons, and PET images are constructed from a collection of Lines of Response (LOR), which are lines connecting the points of interaction and containing the point of emission. In order to obtain good results, clear and accurate PET images are needed.

A significant improvement in Nuclear Medicine was achieved when Computed Tomography (CT) scanners were used together with PET scanners to scan the same object of interest. Whereas the image from the PET scan provides information about the radiotracer uptake, the CT scan provides the 3D density map of the object. PET images are therefore constructed from a collection of Lines of Response which are afterwards corrected with the appropriate attenuation correction factor provided by the CT scan. The superposition of PET and CT images provide a radiologist with a high quality diagnostic image to assess correctly the status of the patient.

One of the main issues around the obtention of accurate PET images is the movement of the patient during the scan period. Even though a patient will try to stay as still a possible, movement of the head are almost inevitable, since a PET scanning process usually takes no less than 10 - 20 minutes.

Different solutions have been developed to improve the quality of the final PET images. Some of these solutions are based on reducing the movement of the patient during the PET scan and include fixing the patient to the PET system e.g. with belts. This may be particularly uncomfortable, especially to claustrophobic patients.

Other solutions based on the identification of the movements of the patients with different types of cameras and further correction of the PET images with the obtained images of cameras have been developed. In-time adjustment of the position of the PET scanner in response to movements of the patients detected by different kinds of sensors are also present in the state of the art.

Although some of these methods may enhance the quality of the PET images, these methods are extremely challenging and may lead to inaccurate correction of the obtained images. The present disclosure intends to provide improvements over prior art obtention of PET images.

### SUMMARY

In a first aspect, a device for use in a PET scanner is provided. The device comprises a base configured to be worn around a head of a subject, wherein the base comprises three or more radioactive point sources which are non-pure positron emitters which simultaneously emit a positron that annihilates into two back-to-back gamma photons with 511 keV and an additional gamma photon of different energy.

In accordance with this aspect, during a PET scan of the brain, in addition to the radioactive tracers previously administered to a patient, the radioactive point sources of the device can be registered. The position of the point sources worn around the head of the patient is measured with the PET scanner, allowing the detection of any movement of the patient wearing the device. The same photon detection systems already present in the PET scanner may be used for this purpose.

By measuring the position of at least three point sources worn around the head of a patient, a change in the position of the head of the patient during the PET scan can be corrected for in the final PET image. With the position of three point sources, a plane may be constructed intersecting with the three point sources. This allows accurate determination of the three dimensional positions of the point sources, and thus of the head of the patient. This thus allows a more precise alignment or superposition of the PET image with a CT image. The final PET image may be corrected with a precision which may be better than 1 mm and the impact caused by the movement of the patient during a PET scan on a final PET image may be significantly reduced.

With more than three point sources, the accuracy may be further improved.

In some examples, the point sources may have an activity of 3 MBq or less, specifically 2 MBq or less, more specifically around 1 MBq. The point sources of the device may have a radioactivity that is significantly lower than the radioactivity of the previously administered tracer and accumulated in the brain of the patient. Exposure of a patient to radiation is thus not significantly increased using devices according to the present disclosure.

In some examples, the non-pure positron emitter may be one of Na22, Sc44, Zr89 and Rb82. In specific examples, the non-pure positron emitter may be Na22 and the additional photon may have a gamma emission of 1274.5 keV. The Na22 isotope may have a stable activity during the PET scan period and its additional gamma photon may be easily distinguishable from the brain emitted photons of 511 keV because of the significant difference in energy level.

In some examples, the base may be a belt, strip or band. In some examples, the base may be a helmet. In examples, the base may be made of a substantially elastic material to adapt to the head of the subject e.g. resembling a swimming cap or helmet. In preferred examples, the base can be adapted to different shapes and sizes of heads, and remains in the same position around the head of the subject throughout a procedure, involving e.g. a CT scan and a PET scan. In preferred examples, the device may be relatively easy to place to a head of a patient and comfortable to wear.

In another aspect of the present disclosure, a controller is provided. The controller is configured to receive data from one or more photon detectors, to identify sensor events in the photon detectors involving two back-to-back gamma photons of 511 keV and an additional gamma photon of different energy emitted from radioactive point sources on a base worn around a head of the patient. The controller is further configured to determine positions of the radioactive point sources based on the sensor events.

Sensor events may herein be regarded as events detected in the photon detectors of the PET scanner which are caused by radioactive decay of the point sources on the device worn by the subject. The controller may further be able to distinguish between sensor events and diagnostic events. Diagnostic events may herein be regarded as events detected which are caused by radioactive decay of a tracer previously administered to the subject. The controller may thus distinguish between gamma photons emitted from the brain of the patient and gamma photons emitted from the device around the head of the patient.

In some examples, the controller may be configured to receive data from the photon detectors during intervals of 1.5 seconds. In other examples, the controller may be configured to receive data from the PET scan during intervals of 1 second, preferably 0.5 seconds. The position of the patient may be monitored in quasi real time, and the impact of the movements of the patients on the final PET image may be drastically reduced.

In some examples, the controller may be configured to construct a plane with the data obtained from the point sources. In further examples, the plane may be a circumference plane and the controller may be further configured to obtain a centre of the circumference plane. With a minimum of three point sources, a circumference plane may be constructed to accurately correct the body movements of the patient. The centre of the circumference plane may be predicted and the 3D position of the three point sources may be obtained with a precision of less than 1 mm.

In some examples, the controller may be further configured to superpose the final PET image with a CT image. Improved alignment between the PET and CT images may be obtained and a 3D density map of the brain of the patient comprising information about the radiotracer uptake may be successfully provided.

The "controller" as used throughout the present disclosure may refer to hardware, firmware and/or software or combinations thereof. The controller may be provided as part of a control system of a PET scanner, as an add-on or update (e.g. software update) to an existing control system of a PET scanner, or also e.g. as a stand-alone system that may be coupled to a PET scanner, or may wirelessly receive data from the PET scanner.

In a further aspect of the present disclosure, a PET scanner is provided. The PET scanner comprises one or more photon detectors and an example of the control system of the previous aspect.

In some examples, the PET scanner may comprise an inner group of photon detectors, and an outer group of photon detectors radially surrounding the inner group of photon detectors, wherein the inner group of detectors comprises a semiconductor material configured to promote photon scattering, optionally silicon, and wherein the outer group of photon detectors comprises a semiconductor material configured to promote photon absorption, optionally CdTe or CdZnTe. WO 2018/019941 discloses such a PET scanner. The methods described therein may be used to provide a quick and accurate determination of the positions and movements of the point sources (and thus of the head of the subject).

In examples, the controller may be configured to determine the positions of the radioactive point sources using Line-of-Response calculation and Compton cone calculation.

In some examples, a CT image from a separate CT scanner may be used and matched to images of the PET scan. In some examples, the PET scanner may be a wearable PET scanner. Such a PET scanner may be more comfortable for patients. At the same time, a drawback in the prior art is that images previously obtained from a separate CT scan can be linked only with difficulty to the images of the PET scanner, since inevitably the position of the head of the patient will be different when images are taken in separate scanner.

In a further aspect of the present disclosure, a method for monitoring movements of a head of a subject in a PET scanner is provided, wherein the subject wears any example of a device as hereinbefore described around his/her head. The method comprises identifying sensor events in photon detectors involving two back-to-back gamma photons of 511 keV and an additional gamma photon of different energy emitted from the radioactive point sources on the device worn around the head of the subject and determining positions of the radioactive point sources based on the sensor events. The method further comprises deriving movements of the head of the subject from the positions of the radioactive point sources.

In yet a further aspect, a method for scanning a brain of a subject is provided. The subject has been previously administered a radioactive tracer, and any example of a device as hereinbefore disclosed has been arranged around the head of the subject. The method comprises identifying sensor events in photon detectors involving two back-to-back gamma photons of 511 keV and an additional gamma photon of different energy emitted from the radioactive point sources on the device worn around the head of the subject and identifying diagnostic events of two back-to-back gamma photons of 511 keV emitted from the radioactive tracer within the brain of the subject. The method further comprises distinguishing diagnostic events from sensor events and determining positions of the radioactive point sources based on the sensor events. The method also comprises deriving movements of the head of the subject from the positions of the radioactive point sources.

### BRIEF DESCRIPTION OF THE DRAWINGS

Non-limiting examples of the present disclosure will be described in the following, with reference to the appended figures, in which:
Figure 1A shows a device worn around a head of a patient according to an example of the present disclosure;
Figure 1B shows a patient wearing the device of figure 1A in a PET machine according to an example of the present disclosure;
Figure 2 shows a reconstructed image of six point sources and a brain according to an example of the present disclosure;
Figure 3A shows a graph showing a line profile passing through two point sources of figure 2;
Figure 3B shows another graph showing a line profile passing through two point sources of figure 2; and
Figure 4 is a flow chart of a method for monitoring movements of a head of a patient during a PET scan.

The figures refer to example implementations and may only be used as an aid for understanding the claimed subject matter, not for limiting it in any sense.

### DETAILED DESCRIPTION OF EXAMPLES

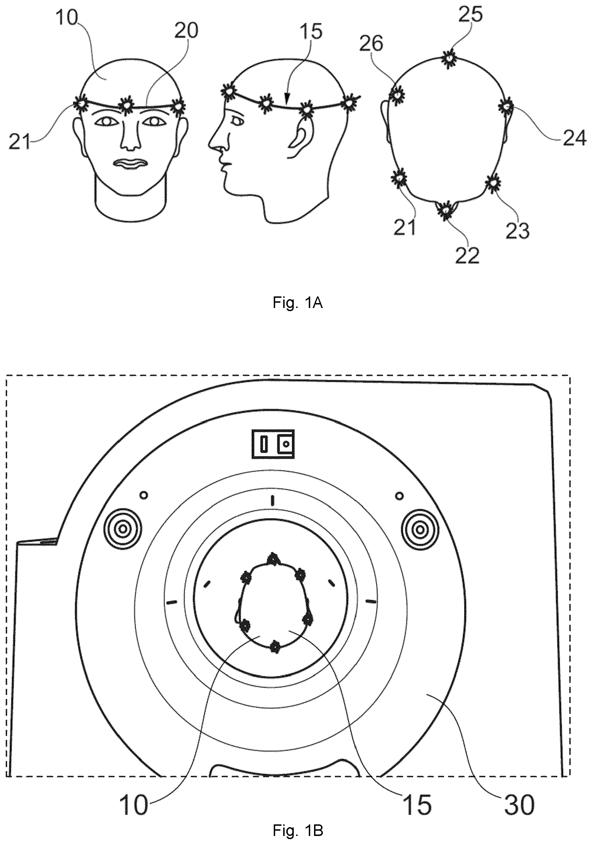

Figure 1A schematically shows a device for a PET scanner according to an example of the present disclosure in a front view, side and top view respectively. Figure 1B shows a patient wearing the device during a PET scan.

The device 15 comprises three or more point sources 21 - 26 configured to be worn around a head 10 of a patient. Each point source 21 - 26 comprises a non-pure positron emitter that simultaneously emits a positron that annihilates into two back-to-back gamma photons with 511 keV and into an additional gamma photon of different energy.

The position of the point sources 21 - 26 worn around the head 10 of the patient may be registered during a PET scan and may be further used to identify whether the position of the head 10 of the patient has changed during the scanning process. With three point sources, a plane of a circumference can be constructed and movements of the patient may be detected and corrected with precision. With more than three point sources, accuracy may be further improved. It should be clear that it is not necessary for all point sources to be arranged in a same plane, when more than three sources are used.

As shown in the example of figure 1A, the base 20 of the device may be configured as a band or belt configured to be worn around the head of the patient during a PET scan. In yet further examples, the base may be configured as a sticker o rplurality of stickers carrying the point sources.

In this particular case, the base 20 may be an elastic, i.e. stretchable belt in this example. The base 20 may include a number of pockets, in which the point sources are arranged. In other examples, the sources may be otherwise attached to the base, e.g. adhesively, with tape or otherwise.

In further non-illustrated examples, the base may be shaped e.g. as a helmet, a hat or cap or any other garment which may be adapted to be attached to or worn by the patient.

The device 15 in this example comprises six sources 21 - 26 which may substantially define a circle. In other examples, the device may comprise a different number of sources, e.g. more than six sensors. The provision of a device comprising more than three point sources positioned around the brain of the patient may allow a more precise detection of the movements of the head of the patient during the PET scan.

The three or more point sources may be separated in order to allow an accurate distinction from the lines of response originated from the different point sources. In some examples, the distance between the sensors may be at least 3 cm.

A radioactive tracer may have been introduced into the circulatory system of the patient before the PET scan to extract information of metabolic activity from the PET images. 18F-FDG is an example of a tracer that is regularly used and is suitable for this purpose. As a result, during the PET scan, and therefore while the patient is wearing the device, the brain of the patient may have an activity in the order of magnitude of 60MBq or more.

In some examples, the point sources of the sensors may be an isotope having an activity of e.g. approximately 1 MBq. The radioactivity of the device may be low enough to be innocuous for the patient but at the same time it may be sufficient to be detected and provide information about the position of the patient during the scan period.

The point sources are a non-pure PET isotope having at least one additional gamma emission in addition to the two back-to-back emitted gamma photons of 511 keV. In some examples, the additional gamma emission may be substantially different from 511 keV such that the PET scanner has enough energy resolution to distinguish between 511 keV and the additional gamma photon.

The non-pure positron emitters may be one of Na22, Sc44, Zr89 and Rb82. In a specific example, the non-pure positron emitters may be Na22 and the additional photon may have a gamma of 1274.5 keV.

The Na22 isotope has a half-lifetime of around 2.6 years which allow the isotope to be bought and stored in advance, and further have a stable activity during the PET scan period. Moreover, the average energy of the emitted e+ is low (250 keV) and this makes the systematics of locating the position of the point source small.

In addition, the location of the point sources may be simplified, as even a PET scanner with poor energy resolution may still distinguish the additional gamma since the energy of the additional gamma of Na22 is 1274.5 keV, which is more than double than 511 keV.

In some examples, the point sources on the base may be substantially identical. In other examples, the point sources may be different e.g. with a different level of activity and or of a different isotopes. Events caused by the decay from different point sources may be distinguished from each other in such examples.

In some examples, the device may also be configured to be imaged in a CT scanner. The position of the point sources may be visible in the CT scan image and the device may thus be used not only to detect any movement of the patient but also to provide co-registration points to align the PET and CT images of the brain of the patient.

In a further aspect of the present disclosure, a controller is provided. The controller is configured to receive data from a PET scan, particularly from photon detectors of the PET scanner. The controller and methods according to the present disclosure may be used with a wide variety of different PET scanners, including existing PET scanners based on semiconductor technology or based on scintillating crystal technology.

The controller may further be configured to identify events involving two back-to-back gamma photons of 511 keV originated from a radiotracer within a brain of a patient and to identify and distinguish events involving two back-to-back gamma photons of 511 keV and an additional gamma photon of different energy emitted from three or more point sources worn around a head of the patient.

The controller is further configured to select data obtained from the point sources and to obtain a 3D position of the point sources. If the 3D position of the point source is the same during the full period of the PET data acquisition, the point sources have not changed position throughout the PET scan and therefore the patient's head did not move. Images of the PET scanner may thus be superposed without difficulty.

On the contrary, if the head of the patient moves during the PET scan, the position of the different point sources located around the head of the patient will not be constant and a change of position of the point sources will be registered.

In some examples, data from the three or more point sources comprises data obtained from the additional gamma photon of different energy emitted by the non-pure positron emitters. Events involving more than the two back-to-back gamma photons may be linked to each other by the timestamp of the events involving an additional gamma ray. In some examples, the controller may be configured to select only the lines of response in coincidence with the emitted additional gamma photon to determine movements of the head of the subject. In a non-limiting example, at least in an initial analysis of the events, only the movement of the head of the subject is registered. Such movements may be sampled every 0,5 seconds, or every second, or every suitable time period. In a final construction of the PET images, the movement of the head of the subject may be combined with the other events corresponding to radioactivity in the brain of the subject.

In some examples, the PET scanner disclosed in WO 2018/019941 may be used. Such a scanner may comprise an inner group of photon detectors, and an outer group of photon detectors radially surrounding the inner group of photon detectors, wherein the inner group of detectors comprises a semiconductor material configured to promote photon scattering, optionally silicon, and wherein the outer group of photon detectors comprises a semiconductor material configured to promote photon absorption, optionally CdTe or CdZnTe.

The inner detectors configured for photon scattering may be any suitable low Z semiconductor material. The outer detectors configured for photon absorption may be any suitable high Z material, such as GaAs, PbS, Hgl2, TIBr, Perovskite and others. In examples, the inner detectors and outer detectors may be arranged on common substrates.

The methods described therein may be used to provide a quick and accurate determination of the positions and movements of the point sources (and thus of the head of the subject). In this case, the controller may be configured to determine the positions of the radioactive point sources using Line-of-Response calculation and Compton cone calculation.

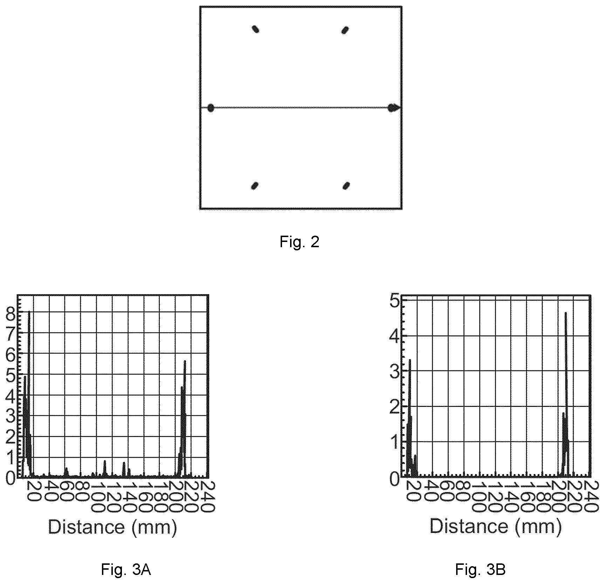

Figure 2 shows an example of a reconstructed image of the head of a patient comprising the device shown in figures 1A and 1B.

The head of the patient may be imaged with any conventional PET scanner. In this particular case, the PET scanner uses LYSO crystals, each 2 mm x 2 mm x 20 mm. The inner radius of the PET scanner may be 210 mm and the axial field of view (FOV) 240 mm. The full width at half maximum (FWHM) defining the energy resolution may be 10% at 511 keV.

In figure 2, six point sources worn around the head of the patient may be identified. The brain of the patient may be located within the illustrated six point sources. In addition, a line profile passing through two of the point sources is represented in the figure.

Figures 3A and 3B are graphics representing data from the line profile passing through the two point sources in figure 2A. The graphics may represent a section of the head of the patient from a first point source e.g. at the right side of the graph to a second point source, opposite the first one e.g. at the left side of the graph. In this example, the distance between the two point sources may be around 200 mm and the brain of the patient may be located within the first and second point source. As a non-limiting example, the two point sources may correspond to sources 21 and 23 of figure 1A, or e.g. as sources 24 and 26 of figure 1A.

In the example of figure 3A, data corresponding to the emission of two back-to-back gamma photons of 511 keV is represented.

In figure 3A, two peaks may be identified at each side of the graphic representing each one of the two point sources. As previously discussed, both the brain of the patient who has been previously administered a radioactive tracer and the point sources may provide data which corresponds to two back-to-back gamma photons of 511 keV. Therefore, additional peaks, located in the graph between the peaks representing the point sources, may be found in the brain of the patient due to the radiotracers present therein.

On the other hand, the example of figure 3B shows a graph in which only the data which coincides with the emission of two back-to-back gamma photons of 511 keV and an additional gamma photon e.g. of 1274.5 keV is represented.

As may be seen, two peaks may be identified in the graphic of figure 3B, representing each one of the two point sources worn by the patient. However, as shown in the example of figure 3B, there is no further data in between the peaks of the two point sources.

Thus, the coincidence with the additional gamma photon e.g. 1274.5 keV may allow to distinguish the lines of response originated from the device around the head of the patient from the ones originated from the radiotracer present in the brain.

Further, when comparing the two peaks of figure 3A and the two peaks of the point sources of figure 3B, it may be seen that the data is not the same. When data corresponding to the emission of two back-to-back gamma photons of 511 keV is used, a full image needs to be constructed in order to locate the point sources worn by the patient, that is, an image of both the brain of the patient and the point sources worn around the head of the patient. However, when the data coming specifically from the point sources is used, only the data corresponding with the emission of two back-to-back gamma photons of 511 keV and the additional gamma photon of 1274.5 keV has to be reconstructed in order to see the change of position of the point sources with respect to a previous position, e.g. every 0.5, 1.0 or 1.5 seconds.

It has been found that the position of the point sources may be more precisely located when filtering the overall data with data comprising two back-to-back gamma photons of 511 keV and an additional gamma photon. The tracking of the positions of the point sources worn around the head of the patient is achieved in a simpler and easier manner, avoiding the need to separate the data which is coming from the brain rather from the non-pure positron emitter point sources and therefore avoiding noise which may inevitably affect the final result.

In some examples, the controller may be configured to receive data from the PET scan in order to determine positions of the sources during intervals of 1.5 seconds, preferably 1 second, more preferably 0.5 seconds and the movements of the patient may be monitored in quasi real time.

Each point source worn around the head of the patient may be positioned at the edge of the scanner ring and, since the spatial resolution may not be the most advantageous (generally the further it is from the centre of the PET scanner, the more it deteriorates), the measured position of the different point sources may be wrong by a few millimetres. To overcome this, the controller may be configured to construct a plane with the position of the point sources. Accordingly, a plane may be constructed to accurately correct the change of position of the patient.

In some examples, the plane may be the plane of a circumference. In addition, the controller may be configured to obtain a centre of the circumference plane in X, Y and Z.

In some examples, data may be collected over intervals of 0.5 seconds and each point source may have a diameter of 1 mm and an activity of 1 MBq. The PET images of at least three point sources on the circumference of a circle e.g. with a radius of 120 mm, may be able to predict the centre of the circle with a precision better than 0.4 mm in X, Y and Z.

In order to check the precision of the estimates of the controller, the position of the point sources was calculated.

As schematically shown in figure 2, the patient may be wearing six point sources of Na22 which, in this particular first example, may be located on a circle of centre 0, 0, 0.

In a first step, two back-to-back gamma photons with 511 keV and the additional gamma photon of 1274.5 keV were identified. In a second step, the same procedure was carried out with only two back-to-back gamma photons with 511 keV.

This process was repeated in a second example for a circle with a centre 0, 0, 1, wherein in a first step two back-to-back gamma photons with 511 keV and the additional gamma photon of 1274.5 keV were identified, and in a second step, only two back-to-back gamma photons with 511 keV were identified.

The results may be shown in the following tables 1 and 2.

**Table 1.**

| **EXAMPLE 1** | | | |
|---|---|---|---|
| | **Centre of circle** | | |
| **Real** | **0** | **0** | **0** |
| **511 keV + 511 keV** | 0.0317 | 0.3829 | -0.1053 |
| **511 keV + 511 keV + 1274.5 keV** | 0.0289 | 0.0647 | -0.0201 |

**Table 2.**

| **EXAMPLE 2** | | | |
|---|---|---|---|
| | **Centre of circle** | | |
| **Real** | **0** | **0** | **1** |
| **511 keV + 511 keV** | 0.0456 | 0.4669 | 1.0401 |
| **511 keV + 511 keV + 1274.5 keV** | 0.0655 | 0.1041 | 1.0087 |

Table 1 and table 2 show that the centre of the circle calculated using the data obtained from the two back-to-back gamma photons of 511 keV and the additional gamma photon of 1274.5 keV is closer to the real value both in Example 1 and in Example 2 than when it is calculated using only the emitted two back-to-back gamma photons with 511 keV.

Thus, similarly to the previous results in figures 3A and 3B, tables 1 and 2 show that a better precision of the position of the patient with respect to the PET scan may be obtained when using only the data which coincide with the 1274.5 keV photons i.e. the data obtained exclusively from the non-pure positron emitters of the sensors worn around the head of the patient.

After obtaining the centre of the plane of a circumference quasi in real time, the controller may be configured to correct head movements of the patient and generate a final PET image. Further, the final PET image may be superposed with a CT image of the brain of the patient which may also comprise the point sources. A highly accurate superposed image comprising PET and CT images of the brain of the patient may be provided.

In other examples, the head of the patient may be measured with a PET scanner based on pixel Cd(Zn)Te detector module. Such PET scanner may have excellent energy and spatial resolution and may be able to use Compton Cone - LOR (CCXLOR) intersection for those decays coming from a non-pure positron emitter to determine the precision of the point source with a precision of less than 1 mm on a time scale of 1 second. Examples of suitable detectors for PET scanners may be found e.g. in WO 2010/034619 A1.

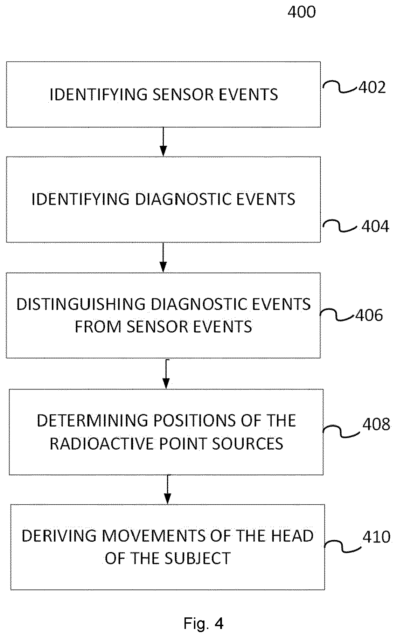

In a further aspect, a method 400 for monitoring movements of a head of a patient during a PET scan is provided. Figure 4 shows a flowchart of the method. The method may comprise arranging around the head of the patient a device with three or more sources, wherein each of the sources comprise a non-pure positron emitter point source that simultaneously emits a positron that annihilates into two back-to-back gamma photons with 511 keV and into an additional gamma photon of different energy.

The method further comprises, at block 402, identifying sensor events in photon detectors involving two back-to-back gamma photons of 511 keV and an additional gamma photon of different energy emitted from the radioactive point sources on the device worn around the head of the patient.

The method further comprises, at block 408, determining positions of the radioactive point sources based on the sensor events, and at block 410, deriving movements of the head of the subject from the positions of the radioactive point sources.

The method may further comprise identifying sensor events in photon detectors from the radioactive point sources on the device worn around the head of the patient, at block 404, identifying diagnostic events emitted from the radioactive tracer within the brain of the patient and, at block 406, distinguishing diagnostic events from sensor events.

Even though the blocks are represented in figure 4 as sequential steps, it should be clear that no specific order of steps is necessary or implied. Similarly, it should be clear that the method may be carried out continuously, and it is not necessary to complete one cycle of blocks/steps before starting another cycle.

The method may comprise scanning a brain of the patient with a PET scanner and identifying a position of two back-to-back gamma photons of 511 keV emitted from a radiotracer within the brain of the patient.

The method may also comprise identifying a position of two back-to-back gamma photons of 511 keV and an additional gamma photon of different energy emitted from the three or more point sources worn around the head of the patient, selecting only the data obtained from the point sources, and obtaining a 3D position of the point sources.

Before a PET scan, the circulatory system of the patient may be provided with a radiotracer. Generally, the dose of the radiotracer administered is of 5 MBq/kg, and around a 20% of that dose reaches the brain of the patient. Accordingly, an average adult patient may be administered a radiotracer dose of e.g. around 300 MBq and during the PET scan, an average brain activity of the patient may be 60 MBq. In addition, each point source may have an activity of 1 MBq. The radiotracers within the brain of the patient may emit two back-to-back gamma photons of 511 keV, which may be detected by the PET scanner.

In some examples, selecting only the data obtained from the point sources may comprise identifying and selecting lines of response in coincidence with the emitted additional gamma photon.

In some examples, obtaining a 3D position of the point sources may comprise constructing a circumference plane using the lines of response in coincidence with the emitted additional gamma photon, and obtaining a centre of the circumference plane.

The method may comprise obtaining a CT image of the head of the patient comprising the device comprising the three or more non-pure positron emitter radioactive point sources i.e. obtaining a CT image in which the point sources are visible. For obtaining the CT image, on the head of the patient, the point sources may be positioned in the same place as they will be positioned during the PET scan.

In some examples, the CT image may be obtained before the PET scan. In other examples, the CT image may be obtained after the PET scan. In some examples, the CT images may be obtained in the same CT/PET scanner. In other examples, the CT image(s) may be obtained in a separate device.

The method may further comprise aligning positions of the radioactive point sources during the scanning of the brain of the patient with the positions of the radioactive point sources in the CT image i.e. aligning the lines of response of the point sources obtained with the PET scanner with lines of response of the point sources in the CT image, and superposing the obtained images with CT images obtained with a CT scanner.

In some examples, aligning the lines of response may comprise aligning the position of two back-to-back gamma photons of 511 keV and an additional gamma photon of different energy emitted from the three or more point sources worn around the head of the patient with the lines of response of the point sources in the CT image.

The CT image comprising the three or more sensors may be later used for aligning the CT scan of the brain with the PET scan of the brain and for further attenuation correction for all the data which has been accumulated during the PET scan.

Examples of the methods disclosed herein may be implemented with hardware, software, firmware and combinations thereof. Whether such functionality is implemented as hardware or software depends upon the particular application and design constraints imposed on the overall system. Skilled artisans may implement the described functionality in varying ways for each particular application.

The various illustrative logical blocks, modules, and circuits described in connection with the disclosure herein may be implemented or performed with one or more general-purpose processors, a digital signal processor (DSP), cloud computing architecture, an application specific integrated circuit (ASIC), a field programmable gate array (FPGA) or other programmable logic device, discrete gate or transistor logic, discrete hardware components, or any combination thereof designed to perform the functions described herein. A general-purpose processor may be a microprocessor, but in the alternative, the processor may be any conventional processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration.

For reasons of completeness, various aspects of the present disclosure are set out in the following numbered clauses:
Clause 1. A device for use in a PET scanner, comprising:
   a base configured to be worn around a head of a subject, wherein
   the base comprises three or more radioactive point sources which are non-pure positron emitters which simultaneously emit a positron that annihilates into two back-to-back gamma photons with 511 keV and an additional gamma photon of different energy.
Clause 2. The device of clause 1, wherein the radioactive point sources have an activity of 3 MBq or less, specifically 2 MBq or less, more specifically around 1 MBq.
Clause 3. The device of clause 1 or 2, wherein the non-pure positron emitter is one of Na22, Sc44, Zr89 and Rb82.
Clause 4. The device of any of clauses 1 - 3, wherein the base is a belt, strip or band and/or wherein the base comprises one or more stickers.
Clause 5. The device of any of clauses 1-3, wherein the base is a helmet or cap.
Clause 6. The device of any of clauses 1-5, wherein the base is made of a substantially elastic material to adapt to the head of the subject.
Clause 7. The device of any of clauses 1-6, comprising six radioactive point source arranged substantially defining a circle when worn around the head of the subject.
Clause 8. The device of any of clauses 1-7, wherein a distance between the radioactive point sources is at least 3 cm.
Clause 9. The device of any of clauses 1- 8, wherein the device is further configured to be imaged in a CT scan.
Clause 10. A controller configured to:
   receive data from one or more photon detectors;
   identify sensor events in the photon detectors involving two back-to-back gamma photons of 511 keV and an additional gamma photon of different energy emitted from radioactive point sources on a base worn around a head of the patient;
   determine positions of the radioactive point sources based on the sensor events.
Clause 11. The controller of clauses 10, further configured to construct a plane intersecting with the radioactive point sources.
Clause 12. The controller of clause 10 or 11, wherein the controller is configured to receive data from the photon detectors during intervals of 1.5 seconds, preferably 1 second, more preferably 0.5 seconds.
Clause 13. The controller of any of clauses 10 - 12, wherein the controller is further configured to
   identify diagnostic events of two back-to-back gamma photons of 511 keV emitted from a radiotracer within a brain of a patient; and to
   distinguish diagnostic events from sensor events.
Clause 14. The controller of clause 13, wherein the controller is further configured to generate a final PET image based on the diagnostic events, and to correct for head movements of the subject based on the sensor events.
Clause 15. The controller of clause 14, wherein the controller is further configured to superpose the final PET image with a CT image.
Clause 16. The controller of clause 14, wherein the CT image is received by the controller from a separate CT scanner.
Clause 17. A PET scanner comprising one or more photon detectors and the control system of any of clauses 10 - 16.
Clause 18. The PET scanner of clause 17, wherein the PET scanner comprises an inner group of photon detectors, and an outer group of photon detectors radially surrounding the inner group of photon detectors, wherein
   the inner group of detectors comprises a semiconductor material configured to promote photon scattering, optionally silicon, and wherein
   the outer group of photon detectors comprises a semiconductor material configured to promote photon absorption, optionally CdTe or CdZnTe.
Clause 19. The PET scanner of clause 18, wherein the controller is configured to determine the positions of the radioactive point sources using Line-of-Response calculation and Compton cone calculation.
Clause 20. The PET scanner of any of clauses 17 - 19, wherein the PET scanner is a wearable PET scanner.
Clause 21. The PET scanner of any of clauses 17 - 19, wherein the PET scanner is a PET/CT scanner.
Clause 22. A system for imaging metabolic activity in a head of a subject comprising the PET scanner of any of clauses 17 - 20 and a separate CT scanner.
Clause 23. A method for monitoring movements of a head of a subject in a PET scanner, wherein the device of any of clauses 1 - 9 has been arranged around the head of the subject, the method comprising:
   identifying sensor events in photon detectors involving two back-to-back gamma photons of 511 keV and an additional gamma photon of different energy emitted from the radioactive point sources on the device worn around the head of the subject;
   determining positions of the radioactive point sources based on the sensor events; and
   deriving movements of the head of the subject from the positions of the radioactive point sources.
Clause 24. The method of clause 23, wherein the positions of the radioactive point sources are obtained in intervals of 1.5 seconds, specifically 1 second, more specifically 0.5 seconds.
Clause 25. A method for scanning a brain of a subject, wherein the subject has been previously administered a radioactive tracer, and wherein the device of any of clauses 1 - 9 has been arranged around the head of the subject, the method comprising:
   identifying sensor events in photon detectors involving two back-to-back gamma photons of 511 keV and an additional gamma photon of different energy emitted from the radioactive point sources on the device worn around the head of the subject;
   identifying diagnostic events of two back-to-back gamma photons of 511 keV emitted from the radioactive tracer within the brain of the subject;
   distinguishing diagnostic events from sensor events;
   determining positions of the radioactive point sources based on the sensor events; and
   deriving movements of the head of the subject from the positions of the radioactive point sources.
Clause 26. The method of clause 25, further comprising:
   obtaining a CT image of the head of the patient, the CT image also including the radioactive point sources of the device of any of claims 1 - 9;
   aligning positions of the radioactive point sources during the scanning of the brain of the subject with the positions of the radioactive point sources in the CT image; and
   superposing a final PET image with the CT image.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow.

## Claims

1. A device (15) for use in a PET scanner, comprising:
a base configured to be worn around a head of a subject, wherein
the base comprises three or more radioactive point sources (21, 22, 23, 24, 25, 26) which are non-pure positron emitters which simultaneously emit a positron that annihilates into two back-to-back gamma photons with 511 keV and an additional gamma photon of different energy.

2. The device (15) of claim 1, wherein the radioactive point sources have an activity of 3 MBq or less, specifically 2 MBq or less, more specifically around 1 MBq.

3. The device (15) of claims 1 or 2, wherein the non-pure positron emitter is one of Na22, Sc44, Zr89 and Rb82.

4. The device (15) of any of claims 1-3, wherein the base is a belt, strip or band.

5. The device (15) of any of claims 1-4, comprising six radioactive point sources (21, 22, 23, 24, 25, 26) arranged substantially defining a circle when worn around the head of the subject.

6. A controller configured to:
receive data from one or more photon detectors;
identify sensor events in the photon detectors involving two back-to-back gamma photons of 511 keV and an additional gamma photon of different energy emitted from radioactive point sources on a base worn around a head of a subject;
determine positions of the radioactive point sources based on the sensor events.

7. The controller of claim 6, further configured to construct a plane intersecting with the radioactive point sources.

8. The controller of any of claims 6 - 7, wherein the controller is further configured to
identify diagnostic events of two back-to-back gamma photons of 511 keV emitted from a radiotracer within a brain of a patient; and to
distinguish diagnostic events from sensor events.

9. The controller of claim 8, wherein the controller is further configured to generate a final PET image based on the diagnostic events, and to correct for head movements of the subject based on the sensor events.

10. A PET scanner comprising one or more photon detectors and the controller of any of claims 6 - 9.

11. The PET scanner of claim 10, wherein the PET scanner comprises an inner group of photon detectors, and an outer group of photon detectors radially surrounding the inner group of photon detectors, wherein
the inner group of detectors comprises a semiconductor material configured to promote photon scattering, optionally silicon, and wherein
the outer group of photon detectors comprises a semiconductor material configured to promote photon absorption, optionally CdTe or CdZnTe.

12. The PET scanner of claim 11, wherein the controller is configured to determine the positions of radioactive point sources using Line-of-Response calculation and Compton cone calculation.

13. A system for imaging metabolic activity in a head of a subject comprising the PET scanner of any of claims 10 - 12 and a separate CT scanner.

14. A method (400) for monitoring movements of a head of a subject in a PET scanner, wherein the device (15) of any of claims 1 - 5 has been arranged around the head of the subject, the method comprising:
identifying sensor events (402) in photon detectors involving two back-to-back gamma photons of 511 keV and an additional gamma photon of different energy emitted from the radioactive point sources on the device worn around the head of the subject;
determining positions (408) of the radioactive point sources based on the sensor events; and
deriving movements (410) of the head of the subject from the positions of the radioactive point sources.

15. A method for scanning a brain of a subject, wherein the subject has been previously administered a radioactive tracer, and wherein the device (15) of any of claims 1-5 has been arranged around the head of the subject, the method comprising:
identifying sensor events in photon detectors involving two back-to-back gamma photons of 511 keV and an additional gamma photon of different energy emitted from the radioactive point sources on the device worn around the head of the subject;
identifying diagnostic events of two back-to-back gamma photons of 511 keV emitted from the radioactive tracer within the brain of the subject;
distinguishing diagnostic events from sensor events;
determining positions of the radioactive point sources based on the sensor events; and
deriving movements of the head of the subject from the positions of the radioactive point sources.
